(19) **Europäisches Patentamt / European Patent Office / Office européen des brevets**

(11) **EP 3 775 917 B1**

(12) # EUROPEAN PATENT SPECIFICATION

(45) Date of publication and mention of the grant of the patent:
**07.01.2026  Bulletin 2026/02**

(21) Application number: **19715503.9**

(22) Date of filing: **10.04.2019**

(51) International Patent Classification (IPC):
***G01N 33/68*** *(2006.01)*

(52) Cooperative Patent Classification (CPC):
**G01N 33/6803; G01N 33/6893;** G01N 2333/47;
G01N 2800/18

(86) International application number:
**PCT/EP2019/059041**

(87) International publication number:
**WO 2019/197444 (17.10.2019 Gazette 2019/42)**

(54) **GINGIVITIS DIAGNOSTIC METHODS, USES AND KITS**

GINGIVITIS-DIAGNOSEVERFAHREN, VERWENDUNGEN UND KITS

PROCÉDÉS, UTILISATIONS ET KITS DE DIAGNOSTIC DE LA GINGIVITE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **12.04.2018  EP 18166943**

(43) Date of publication of application:
**17.02.2021  Bulletin 2021/07**

(73) Proprietor: **Koninklijke Philips N.V.**
**5656 AG Eindhoven (NL)**

(72) Inventors:
- **KOOIJMAN, Gerben**
 **5656 AE Eindhoven (NL)**
- **RMAILE, Amir, Hussein**
 **5656 AE Eindhoven (NL)**
- **GLASSE, Carl**
 **5656 AE Eindhoven (NL)**
- **DE JAGER, Marinus, Karel, Johannes**
 **5656 AE Eindhoven (NL)**
- **CHAPPLE, Iain, Leslie, Campbell**
 **5656 AE Eindhoven (NL)**
- **GRANT, Melissa, Mackay**
 **5656 AE Eindhoven (NL)**
- **PRESHAW, Philip**
 **5656 AE Eindhoven (NL)**
- **TAYLOR, John**
 **5656 AE Eindhoven (NL)**
- **VAN HARTSKAMP, Michael, Alex**
 **5656 AE Eindhoven (NL)**

(74) Representative: **Philips Intellectual Property & Standards**
**High Tech Campus 52**
**5656 AG Eindhoven (NL)**

(56) References cited:
**WO-A2-2014/037924**

- **MELISSA M. GRANT ET AL: "Proteomic Analysis of a Noninvasive Human Model of Acute Inflammation and Its Resolution: The Twenty-one Day Gingivitis Model", JOURNAL OF PROTEOME RESEARCH., vol. 9, no. 9, 3 September 2010 (2010-09-03), US, pages 4732 - 4744, XP055477184, ISSN: 1535-3893, DOI: 10.1021/pr100446f**
- **L. DA R. GONÇALVES ET AL: "Analysis of the salivary proteome in gingivitis patients : Saliva proteome from gingivitis patients", JOURNAL OF PERIODONTAL RESEARCH, 1 June 2011 (2011-06-01), pages no - no, XP055477525, ISSN: 0022-3484, DOI: 10.1111/j.1600-0765.2011.01378.x**
- **OLE BRINKMANN ET AL: "Salivary biomarkers for periodontal disease diagnostics", EXPERT OPINION ON MEDICAL DIAGNOSTICS, vol. 5, no. 1, 22 January 2011 (2011-01-22), GB, pages 25 - 35, XP055400010, ISSN: 1753-0059, DOI: 10.1517/17530059.2011.542144**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 775 917 B1

- GHALLAB NOHA AYMAN: "Diagnostic potential and future directions of biomarkers in gingival crevicular fluid and saliva of periodontal diseases: Review of the current evidence", ARCHIVES OF ORAL BIOLOGY, vol. 87, 1 March 2018 (2018-03-01), pages 115 - 124, XP085348826, ISSN: 0003-9969, DOI: 10.1016/J.ARCHORALBIO.2017.12.022
- GUY M. ABOODI ET AL: "Salivary Cytoprotective Proteins in Inflammation and Resolution during Experimental Gingivitis-A Pilot Study", FRONTIERS IN CELLULAR AND INFECTION MICROBIOLOGY, vol. 5, 5 January 2016 (2016-01-05), XP055477538, DOI: 10.3389/fcimb.2015.00092
- THIAGO MORELLI ET AL: "Salivary Biomarkers in a Biofilm Overgrowth Model", JOURNAL OF PERIODONTOLOGY., vol. 85, no. 12, 1 December 2014 (2014-12-01), US, pages 1770 - 1778, XP055427552, ISSN: 0022-3492, DOI: 10.1902/jop.2014.140180

**Description**

FIELD OF THE INVENTION

The invention is in the field of oral care, and pertains to saliva-based diagnostics of periodontal disease. Particularly, the invention pertains to a kit, use and method for diagnosing gingivitis.

BACKGROUND OF THE INVENTION

Gum inflammation, or gingivitis, is a non-destructive periodontal disease caused mainly by the adherence of dental bacterial biofilms, or dental plaque, to the tooth surface. If not detected and treated, the reversible gingivitis usually leads to the inflammation of the tissues surrounding the tooth (i.e. periodontal tissues), a condition defined as periodontitis, which is irreversible and causes tissue destruction and alveolar bone loss, and ultimately results in the loss of teeth. During the progression of gum disease, there are usually clinical signs and symptoms associated with it, such as the swelling of the gums, the change in color from pink to dark red, the bleeding of the gums, bad breath, and the gums becoming more tender or painful to touch.

Periodontitis is a chronic multifactorial inflammatory disease caused by oral microorganisms and characterized by progressive destruction of the hard (bone) and soft (periodontal ligament) tissues, ultimately leading to tooth mobility and loss. This is to be distinguished from gingivitis which is a reversible infection and inflammation of the gum tissues. Inflammatory periodontitis is one of the most prevalent chronic human diseases and a major cause of adult tooth loss. In addition to the substantial negative impact of periodontitis on oral health, there is also mounting evidence that periodontitis has systemic consequences and that it is a risk factor for several systemic diseases, including heart diseases (e.g. atherosclerosis, stroke), diabetes, pregnancy complications, rheumatoid arthritis and respiratory infections.

Early and accurate diagnosis of periodontal disease, thus, is important from both an oral and overall health perspective.

Periodontal diseases are still poorly diagnosed in general dental practice, resulting in relatively low rates of therapeutic intervention and significant amounts of untreated cases. Current diagnosis relies on imprecise, subjective clinical examination of oral tissue condition (color, swelling, extent of bleeding on probing, probing pocket depth; and bone loss from oral x-rays) by dental professionals. These conventional methods are time consuming, and some of the techniques used (pocket-depth, x-ray) reflect historic events, such as past disease activity, rather than current disease activity or susceptibility to further disease. Hence, more objective, faster, accurate, easier-to-use diagnostics which preferably may also be performed by non-specialists are desirable. Thereby it is desirable to measure current disease activity, and possibly a subject's susceptibility to further periodontal disease.

Saliva or oral fluids have long been advocated as a diagnostic fluid for oral and general diseases, and with the advent of miniaturized biosensors, also referred to as lab-on-a-chip, point of care diagnostics for rapid chair-side testing have gained greater scientific and clinical interest. Especially for periodontal disease detection, inflammatory biomarkers associated with tissue inflammation and breakdown may easily end up in saliva due to proximity, suggesting saliva has strong potential for periodontal disease detection. Indeed, this area thus has gained significant interest and encouraging results have been presented. For example, Ramseier et al (J Periodontol. 2009 Mar;80(3):436-46) identified host- and bacterially derived biomarkers correlated with periodontal disease. However, no definite test has emerged yet.

WO-A-2014/037924 describes methods for diagnosing the status of periodontitis disease includes selecting a set of protein biomarkers including one or more biomarkers which have been shown to vary in abundance at particular stages of periodontitis.

Biomarkers represent biological indicators that underpin clinical manifestations, and as such are objective measures by which to diagnose clinical outcomes of periodontal disease. Ultimately, proven biomarkers could be utilized to assess risk for future disease, to identify disease at the very earliest stages, to identify response to initial therapy, and to allow implementation of preventive strategies.

Previous limitations to the development of point-of-care tests for salivary biomarkers included a lack of technologies that were adaptable to chair-side applications and an inability to analyze multiple biomarkers in individual samples. Also the selection of which multiple biomarkers to include in such a test has not been adequately addressed in the literature nor implemented in practical tests.

It would be desired to provide a simpler process, and particularly a process that requires only that a small saliva sample is taken from a patient, and possibly by the patient him- or herself. It is desired that such a sample be entered into an *in vitro* diagnostic device, which will allow, based on measurement, a classification of the saliva sample such that it can return an indication of the likelihood that the patient is to be classified as suffering from gingivitis.

SUMMARY OF THE INVENTION

The invention is defined by the claims.

In order to better address the foregoing desires, the invention, in one aspect, concerns an *in vitro* method for assessing whether a human patient has gingivitis, the method comprising detecting, in a sample of saliva from said human patient, the concentrations of the proteins:

Alpha-1-acid glycoprotein (A1AGP), Matrix metalloproteinase-8 (MMP8), Matrix metalloproteinase-9 (MMP9), and Hemoglobin subunit beta (Hb-beta);

determining a testing value reflecting the joint concentrations determined for said proteins; and comparing the testing value with a threshold value reflecting in the same manner the joint concentrations associated with gingivitis, so as to assess whether the testing value is indicative for gingivitis in said patient.

In another aspect, the invention presents the use of the proteins of the first aspect in a saliva sample of a human patient, as biomarkers for assessing whether the patient has gingivitis.

Optionally, the age of the patient is also used as a biomarker.

In a further aspect, the invention resides in a system for assessing whether a human patient has gingivitis, the system comprising:

- detection means able and adapted to detect in a sample of saliva of the human patient the concentration of the proteins:
  Alpha-1-acid glycoprotein (A1AGP), Matrix metalloproteinase-8 (MMP8), Matrix metalloproteinase-9 (MMP9) and Hemoglobin subunit beta (Hb-beta);; and
- a processor able and adapted to determine from the determined concentrations of said proteins an indication of the patient having gingivitis.

The system optionally contains a data connection to an interface, particularly a graphical user interface, capable of presenting information, preferably also capable of putting in information such as the age of the subject, as well as optionally other information such as sex and/or BMI (Body Mass Index), said interface being either a part of the system or a remote interface.

Optionally one or more of the foregoing items, particularly the processor, are enabled to function "in the cloud", i.e., not on a fixed machine, but by means of an internet-based application.

In a still further aspect, the invention provides a kit for detecting at least four biomarkers for gingivitis in a sample of saliva of a human patient, said kit comprising detection reagents for detecting the proteins:

Alpha-1-acid glycoprotein (A1AGP), Matrix metalloproteinase-8 (MMP8), Matrix metalloproteinase-9 (MMP9) and Hemoglobin subunit beta (Hb-beta).

Typically, four or more detection reagents are used, each of which binds a different biomarker.

In a further aspect, the invention provides a method of diagnosing whether a human patient has gingivitis, comprising detecting in a sample of saliva of the human patient the proteins:

Alpha-1-acid glycoprotein (A1AGP) and at least one of Matrix metalloproteinase-8 (MMP8), Matrix metalloproteinase-9 (MMP9), Hepatocyte growth factor (HGF), Hemoglobin subunit beta (Hb-beta), and S100 calcium-binding protein A8 (S100A8);

and assessing the presence of gingivitis in the patient on the basis of the concentrations of said proteins in said sample. Optionally, the method of this aspect comprises the further step of treating the gingivitis in the patient.

BRIEF DESCRIPTION OF THE DRAWINGS

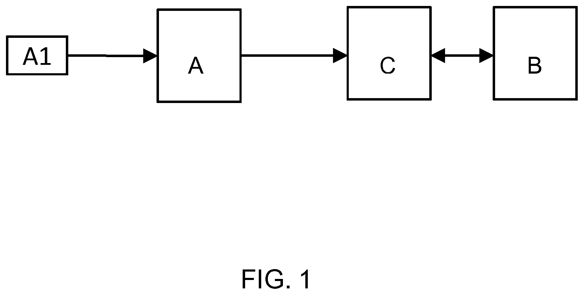

Fig. 1 schematically represents a system for use in the method as described in this disclosure.

DETAILED DESCRIPTION OF THE INVENTION

The invention is defined by the claims.

In a general sense, the invention is based on the judicious insight that gingivitis can be distinguished from a healthy oral cavity with sufficient accuracy based on a measurement of a handful of protein biomarkers. In particular, it has been found that as few as two proteins can serve as a biomarker in a sample of saliva of a human patient, for identifying the presence or absence of gingivitis.

The disclosed biomarker proteins are Alpha-1-acid glycoprotein (A1AGP), Matrix metalloproteinase-8 (MMP8), Matrix metalloproteinase-9 (MMP9), Hepatocyte growth factor (HGF), Haemoglobin subunit beta (Hb-beta), S100 calcium-binding protein A8 (S100A8), Keratin 4 (K-4), Interleukin-1β (IL-1β), and Profilin. The following combination of these proteins are used to diagnose gingivitis according to the invention:

Alpha-1-acid glycoprotein (A1AGP), Matrix metalloproteinase-8 (MMP8), Matrix metalloproteinase-9 (MMP9), and Hemoglobin subunit beta (Hb-beta).

The subject's age may optionally be included as an additional marker.

Alpha-1-acid glycoprotein (A1AGP) is a plasma alpha-globulin glycoprotein synthesized primarily by the liver. It is also sometimes known as Orosomucoid. It functions as a transport protein in the blood acts as a carrier of basic and neutrally charged lipohillic compounds. It is also believed to regulate the interaction between blood cells and endothelial cells.

MMPs are a family of enzymes that are responsible for the degradation of extracellular matrix components such as collagen, proteoglycans, laminin, elastin, and fibronectin. They play a central role in the periodontal ligament (PDL) remodelling, both in physiological and pathological conditions. MMP-8, also known as neutrophil collagenase or PMNL collagenase (MNL-CL), is a collagen protease enzyme which is present in the connective tissue of most mammals. MMP-9, also known as 92 kDa type IV collagenase, 92 kDa gelatinase or gelatinase B (GELB), is a matrixin, a class of enzymes that belong to the zinc-metalloproteinases family involved in the degradation of the extracellular matrix.

Hepatocyte Growth Factor (HGF) is a paracrine cellular growth, motility and morphogenic factor. It is secreted by mesenchymal cells and targets and acts primarily upon epithelial cells and endothelial cells, but also acts on haemopoietic progenitor cells. HGF has been shown to have a major role in myogenesis and in wound healing. Its ability to stimulate mitogenesis, cell motility, and matrix invasion gives it a central role in angiogenesis, tumorogenesis, and tissue regeneration. HGF stimulates growth of epithelial cells and prevents regeneration of the connective tissue attachment. HGF is known as a serum marker indicating disease activity in various diseases.

Haemoglobin (Hb) is the iron-containing oxygen-transport metalloprotein in the red blood cells of nearly all vertebrates as well as the tissues of some invertebrates. Haemoglobin-beta (also known as beta globin, HBB, $\beta$-globin, and haemoglobin subunit beta) is a globin protein, which along with alpha globin (HBA), makes up the most common form of haemoglobin in adult humans, the HbA. Hb-$\beta$ is typically 146 amino acids long and has a molecular weight of 15,867 Da. Normal adult human HbA is a heterotetramer consisting of two alpha chains and two beta chains. Hb-$\beta$ is encoded by the HBB gene on human chromosome 11.

S100 calcium binding protein A8 is a calcium- and zinc-binding protein which plays a prominent role in the regulation of inflammatory processes and immune response. It can induce neutrophil chemotaxis and adhesion.

Keratin-4 (K4), also known as cytoskeletal Keratin 4 (CYK4) or cytokeratin-4 (CK-4) is a protein that in humans is encoded by the KRT4 gene. It is a member of the keratin gene family. The type II cytokeratins consist of basic or neutral proteins which are arranged in pairs of heterotypic keratin chains coexpressed during differentiation of simple and stratified epithelial tissues. The type II cytokeratin CK4 is specifically expressed in differentiated layers of the mucosal and esophageal epithelia with family member KRT13. Mutations in these genes have been associated with White Sponge Nevus, characterized by oral, esophageal, and anal leukoplakia. The type II cytokeratins are clustered in a region of chromosome 12q12-q13.

Interleukin 1-beta (IL-1$\beta$) is a member of the interleukin 1 family of cytokines. This cytokine is produced by activated macrophages as a proprotein, which is proteolytically processed to its active form by caspase 1 (CASP1/ICE). This cytokine is an important mediator of the inflammatory response, and is involved in a variety of cellular activities, including cell proliferation, differentiation, and apoptosis.

Profilin is an actin-binding protein involved in the dynamic turnover and restructuring of the actin cytoskeleton, found in most cells. It is important for spatially and temporally controlled growth of actin microfilaments, which is an essential process in cellular locomotion and cell shape changes. Human profilin-1 is typically 140 amino acids long when expressed but is often further processed into a mature form.

The proteins mentioned above are known in the art. The skilled person is aware of their structure, and of methods to detect them in an aqueous sample, such as a saliva sample. Hereinafter the following protein biomarker combinations are collectively referred to as "the biomarker panels of the invention":

Alpha-1-acid glycoprotein (A1AGP), Matrix metalloproteinase-8 (MMP8), Matrix metalloproteinase-9 (MMP9), and Hemoglobin subunit beta (Hb-beta).

Table 1 in the Example provides 14 particularly preferred combinations according to the disclosure.

A biomarker panel of the invention, in one embodiment, may consist of the protein biomarkers identified. Preferably, a biomarker panel of the invention consists of not more than four of the protein biomarkers identified in the invention, e.g. four protein biomarkers of the invention. In addition to the biomarker panels of the invention, other biomarkers and or data, such as demographic data (e.g., age, sex) can be included in a set of data applied for the determination of the type of gingivitis.

An example of an additional protein biomarker is Free Light Chain Kappa. This is included in some of the preferred biomarker panels in Table 1, below. Free Light Chain proteins are immunoglobulin light chains. They are not associated with an immunoglobulin heavy chain. Unlike a typical whole immunoglobulin molecule, a Free Light Chain protein is not covalently linked to an immunoglobulin heavy chain, e.g. the Free Light Chain is not disulphide bonded to a heavy chain. Typically the Free Light Chain comprises approximately 220 amino acids. Typically, the Free Light Chain protein comprises a variable region (often referred to as the Light Chain variable Region, $V_L$) and a constant region (often referred to as the Light Chain constant Region, $C_L$). Humans produce two types of immunoglobulin light chains, named with the letter kappa ($\kappa$) and lambda ($\lambda$). Each of these can be further divided into sub-groups based on variation in the variable region, with four kappa subtypes (V$\kappa$1, V$\kappa$2, V$\kappa$3 and V$\kappa$4) and six lambda subtypes (V$\lambda$1, V$\lambda$2, V$\lambda$3, V$\lambda$4, V$\lambda$5 and V$\lambda$6). Free Light Chain $\kappa$ is typically monomeric. Free Light Chain $\lambda$ is typically dimeric, linked by disulphide bonding (to another Free Light Chain $\lambda$). Polymeric forms of Free Light Chain $\lambda$ and of Free Light Chain $\kappa$ have been identified. Free light chains are produced by bone marrow and lymph node cells as well as locally in the periodontium by diffuse lymphocytes, and are rapidly cleared from the blood and catabolised by the kidneys. Monomeric free light chains are cleared in 2-4 hours, and dimeric free light chains in 3-6 hours.

When other biomarkers are optionally included, the total number of biomarkers (i.e. the biomarker panel of the invention plus other biomarkers) is typically 5 or 6.

However, a desirable advantage of the present invention is that the classification of gingivitis in a patient can be determined by measuring preferably not more than four biomarkers. Particularly, the determination does not need to involve the use of other data, which advantageously provides a simple and straightforward diagnostic test.

The method, as desired, requires only that a small saliva sample, e.g. a dropsize, is taken from the subject. The size of the sample will typically range of from 0.1 $\mu$l to 2 ml, such as 1-2 ml, whereby smaller amounts, e.g., 0.1 to 100 $\mu$l can be used for *in vitro* device processing, and whereby taking a larger sample, such as up to 20 ml, such as 7.5 to 17 ml, is also possible.

This sample is entered into an *in vitro* diagnostic device, which measures the concentration(s) of the proteins involved, and which returns a diagnostic outcome, classifying the subject on the basis of a likelihood of having gingivitis.

The ease of use of this invention will make it possible to test the majority of dental patients with gingivitis, or with a high risk for developing gingivitis, on a regular basis (e.g. as part of a regular dental check or even at home). This allows, *inter alia*, detecting the presence of gingivitis soon after it has developed, and thus enables more timely taking oral care measures to prevent its progress to periodontitis and to reverse the effects of gingivitis. Or, e.g., with patients known to be at high risk for gingivitis, and tested for the first time, the method allows to identify whether the gingivitis has developed. Particularly, the method is also suitable for self-diagnosis, whereby the steps of taking the sample and entering it into a device can be conducted by the patient him- or herself.

The patient may typically be known or suspected to have gingivitis when the invention is carried out to confirm whether the gingivitis is present. In certain embodiments therefore, the method is for assessing whether a human patient, known or suspected to have gingivitis, has gingivitis. In performing a 'health or gingivitis classification' on a subject, it is already known or assumed that the subject does not suffer from periodontitis. This can either be known from e.g. a previously performed periodontitis detection/classification procedure, or e.g. assumed from the subject's oral health condition record.

A method of the invention typically comprises detecting the aforementioned proteins making up a biomarker panel of the invention, and optional further biomarker proteins, by using one or more detection reagents.

The "saliva" that is tested according to the invention may be undiluted saliva, which may be obtained by spitting or swabbing, or diluted saliva, which may be obtained by rinsing the mouth with a fluid. Diluted saliva may be obtained by the patient rinsing or swilling their mouth for a few seconds with sterile water (for example 5ml or 10ml) or other suitable fluid, and spitting into a container. Diluted saliva may sometimes be referred to as an oral rinse fluid.

By "detecting" is meant measuring, quantifying, scoring, or assaying the concentration of the biomarker proteins. Methods of evaluating biological compounds, including biomarker proteins, are known in the art. It is recognized that methods of detecting a protein biomarker include direct measurements and indirect measurements. One skilled in the art will be able to select an appropriate method of assaying a particular biomarker protein.

The term "concentration" with respect to the protein biomarkers is to be given its usual meaning, namely the abundance of the protein in a volume. Protein concentration is typically measured in mass per volume, most typically mg/ml, $\mu$g/ml or ng/ml, but sometimes as low as pg/ml. An alternative measure is Molarity (or Molar concentration), mol/L or "M". The concentration can be determined by detecting the amount of protein in a sample of known, determined or pre-determined volume.

An alternative to determining the concentration is to determine the absolute amount of the protein biomarker in the sample, or determining the mass-fraction of the biomarker in the sample, for example the amount of the biomarker relative to the total of all other proteins in the sample.

A "detection reagent" is an agent or compound that specifically (or selectively) binds to, interacts with or detects the protein biomarker of interest. Such detection reagents may include, but are not limited to, an antibody, polyclonal antibody, or monoclonal antibody that preferentially binds the protein biomarker.

The phrase "specifically (or selectively) binds" or "specifically (or selectively) immunoreactive with," when referring to a detection reagent, refers to a binding reaction that is determinative of the presence of the protein biomarker in a heterogeneous population of proteins and other biologics. Thus, under designated immunoassay conditions, the specified detection reagent (e.g. antibody) binds to a particular protein at least two times the background and does not substantially bind in a significant amount to other proteins present in the sample. Specific binding under such conditions may require an antibody that is selected for its specificity for a particular protein. A variety of immunoassay formats may be used to select antibodies specifically immunoreactive with a particular protein. For example, solid-phase ELISA immunoassays (enzyme linked immunosorbent assay) are routinely used to select antibodies specifically immunoreactive with a protein (see, e.g., Harlow & Lane, Antibodies, A Laboratory Manual (1988), for a description of immunoassay formats and conditions that can be used to determine specific immunoreactivity). Typically a specific or selective reaction will be at least twice the background signal or noise and more typically more than 10 to 100 times the background.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases. This includes, e.g., Fab' and F(ab)'2 fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region. The antibody may be a bispecific antibody, e.g. an antibody that has a first variable region that specifically binds to a first antigen and a second variable region that specifically binds to a second, different, antigen. Use of at least one bispecific antibody can reduce the number of detection reagents needed.

Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive.

The biomarker protein(s) of the invention can be detected in a sample by any means. Preferred methods for biomarker detection are antibody-based assays, protein array assays, mass spectrometry (MS) based assays, and (near) infrared spectroscopy based assays. For example, immunoassays, include but are not limited to competitive and non-competitive assay systems using techniques such as Western blots, radioimmunoassays, ELISA, "sandwich" immunoassays, immunoprecipitation assays, precipitin reactions, gel diffusion precipitin reactions, immunodiffusion assays, fluorescent immunoassays and the like. Such assays are routine and well known in the art. Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1 % sodium deoxycholate, 0.1% SDS, 0.15 M NaCl, 0.01 M sodium phosphate at pH 7.2, 1 % Trasylol) supplemented with protein phosphatase and/or protease inhibitors (e.g., EDTA, PMSF, aprotinin, sodium vanadate), adding an antibody of interest to the cell lysate, incubating for a period of time (e.g., 1-4 hours) at 4°C, adding protein A and/or protein G sepharose beads to the cell lysate, incubating for about an hour or more at 4°C, washing the beads in lysis buffer and resuspending the beads in SDS/sample buffer. The ability of the antibody to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with Sepharose beads).

Western blot analysis generally comprises preparing protein samples, electrophoresis of the protein samples in a polyacrylamide gel (e.g., 8%-20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 125I) diluted in blocking buffer, washing the membrane in wash buffer, and detecting the presence of the antigen. One of skill in the art would be knowledgeable as to the parameters that can be modified to increase the signal detected and to reduce the background noise.

ELISAs typically comprise preparing antigen (i.e. the biomarker protein of interest or fragment thereof), coating the well of a 96- well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art.

Since multiple markers are used, a threshold is determined on the basis of the joint concentrations of the biomarkers (and optionally age). This threshold determines whether a patient is classified as having gingivitis or not. The invention reflects the insight that gingivitis can be detected, with sufficient accuracy based on a measurement of the combination of biomarkers as indicated above.

This insight supports another aspect, the invention, which is the use of the proteins:

Alpha-1-acid glycoprotein (A1AGP), Matrix metalloproteinase-8 (MMP8), Matrix metalloproteinase-9 (MMP9)and Hemoglobin subunit beta (Hb-beta);

as biomarkers in a saliva sample of a human patient, for assessing whether the patient has gingivitis.

This use can be implemented in a method as substantially described hereinbefore and hereinafter.

The method of the invention comprises determining a testing value reflecting the joint concentrations measured for said proteins. A joint concentration value can be any value obtained by input of the concentrations as determined and an arithmetic operation of these values. This can, e.g., be a simple addition of the concentrations. It can also involve multiplying each concentration with a factor reflecting a desired weight of these concentrations, and then adding up the results. It can also involve multiplying the concentrations with each other, or any combination of multiplication, division, subtraction, exponentiation, and addition. It can further involve raising concentrations to some power.

Optionally, the testing value reflects the concentration of joint concentrations determined for said protein(s) in combination with the age of the subject.

The resulting joint concentration value is compared with a threshold value reflecting in the same manner the joint concentrations associated with the presence of gingivitis. The comparison allows assessing whether the testing value is indicative of the presence of gingivitis in the patients whose saliva is subjected to the test.

The threshold value can, e.g., be based on the joint concentration value, obtained in the same manner on the basis of the concentration(s) determined for the same protein(s) in a reference sample associated with the presence of gingivitis, i.e. in a patient diagnosed with gingivitis. Typically, thereby a value reflecting the same or higher joint concentration is indicative of the presence of gingivitis in a tested patient. Analogously, a value reflecting a lower joint concentration in the saliva of a tested gingivitis patient, indicates that gingivitis is absent. However, it will be understood that it is also possible to calculate a threshold value (e.g. by using a negative multiplier) such that a testing value indicating gingivitis would be below the threshold, and a testing value indicating absence of gingivitis, would be above the threshold.

The threshold value can also be determined on the basis of measuring the concentration(s) of the present biomarker protein(s) in a set of samples, including patients with a known diagnosis of gingivitis and "not" gingivitis. Thereby the measured concentration values can be subjected to statistical analysis, possibly including machine learning methods, allowing to discriminate, with the desired sensitivity and specificity, patients classified as gingivitis and patients classified as not suffering from gingivitis. Therefrom, the desired threshold value can be obtained. On the basis of this threshold value, a sample to be tested can be subjected to the same concentration measurement, and the concentration values are then processed, in the same manner in which the threshold value is obtained, so as to determine a joint concentration value that can be compared with the threshold, thus allowing the tested sample to be classified as having gingivitis or not.

In an interesting embodiment, the joint concentration value is obtained in the form of a score as follows. A numerical value (protein concentration values in e.g. ng/ml) is assigned to each measurement, and these values are used in a linear or non-linear combination to calculate a score between zero and one. In the event that the threshold value is determined on the basis of a set of subjects as mentioned above, the score between 0 and 1 is typically calculated with the sigmoid function that takes the joint concentration as input (as shown further on).

When the score exceeds a certain threshold, the method indicates that the patient has gingivitis. The threshold may be chosen based on the desired sensitivity and specificity.

It will be understood that in performing a 'gingivitis classification' on a subject, in accordance with the invention, this can be on subjects for which there is no knowledge or awareness of their gingivitis status, or on subjects that can be assumed to be at risk from, or suffering from, gingivitis. This prior knowledge can typically either be known from e.g. a previously performed diagnosis of gingivitis, though perhaps without ability to differentiate the extent of it, or, e.g., assumed from the subject's oral health condition record.

Clinical definitions as acknowledged in the art are based on the following:

*Gingival Index (GI)*

A full mouth gingival index will be recorded based on the Lobene Modified Gingival Index (MGI) rated on a scale of 0 to 4, where:

- 0 = absence of inflammation,
- 1 = mild inflammation; slight change in color little change in texture of any portion of but not the entire margin or papillary gingival unit,
- 2 = mild inflammation; but involving entire margin or papillary unit,
- 3 = moderate inflammation; glazing, redness, oedema and/or hypertrophy of margin or papillary unit,
- 4 = severe inflammation; marked redness, oedema and/or hypertrophy of marginal or papillary gingival unit, spontaneous bleeding, congestion, or ulceration].

*Probing depths (PD)*

Probing depths will be recorded to the nearest mm using a manual UNC-15 periodontal probe. Probing depth is the distance from the probe tip (assumed to be at the base of the pocket) to the free gingival margin.

*Gingival recession (REC)*

Gingival recession will be recorded to the nearest mm using a manual UNC-15 periodontal probe. Gingival recession is the distance from the free gingival margin to the cemento-enamel junction. Gingival recession will be indicated as a positive number and gingival overgrowth will be indicated as a negative number.

*Clinical attachment loss (CAL)*

Clinical attachment loss will be calculated as the sum of probing depth + recession at each site.

*Bleeding on probing (BOP)*

Following probing, each site will be assessed for bleeding on probing, if bleeding occurs within 30s of probing, a score of 1 will be assigned for the site, otherwise a score of 0 will be assigned.
The resulting subject group (patient group) definition is as follows, whereby the mild-moderate periodontitis and the advanced periodontitis groups are "periodontitis" relevant to the present invention:

- Healthy group (H): PD $\leq$ 3 mm in all sites (but would allow up to four 4 mm pockets at distal of last standing molars), no sites with interproximal attachment loss, GI of $\geq$ 2.0 in $\leq$ 10% sites, %BOP scores $\leq$ 10%;
- Gingivitis group (G): GI $\geq$ 3.0 in > 30% of sites, no sites with interproximal attachment loss, no sites with PD > 4 mm , % BOP scores > 10%;
- Mild-moderate periodontitis group (MP): interproximal PD of 5-7 mm, (equating to approximately 2-4 mm CAL) at $\geq$ 8 teeth, %BOP scores > 30%;
- Advanced periodontitis group (AP): interproximal PD of $\geq$ 7 mm, (equating to approximately $\geq$ 5 mm CAL) at $\geq$ 12 teeth, %BOP scores > 30%.

In an embodiment, the method of the invention makes use of a system as represented schematically in Fig. 1. The system can be a single apparatus having various device components (units) integrated therein. The system can also have its various components, or some of these components, as separate apparatuses. The components shown in Fig. 1 are a measurement device (A), a graphical user interface (B) and a computer processing unit (C).
As mentioned above, the system of the invention comprises a data connection to an interface, whereby the interface itself can be a part of the system or can be a remote interface. The latter refers to the possibility to use a different apparatus, preferably a handheld apparatus such as a smartphone or a tablet computer, for providing the actual interface. The data connection in such cases will preferably involve wireless data transfer such as by Wi-Fi or Bluetooth, or by other techniques or standards.
The measurement device (A) is configured to receive a saliva sample, for example by putting a drop of saliva on a cartridge (A1), which can be inserted into the device (A). The device can be an existing device that is capable to determine, from the same saliva sample, the concentrations of the proteins.
The processing unit (C) receives numerical values for the protein concentrations from part (A). The unit (C) is provided with software (typically embedded software) allowing it to calculate a score (S) between 0 and 1. The software further includes a numerical value for the threshold (T). If the calculated value (S) exceeds (T), unit (C) will output an indication (I) of 'gingivitis' to the GUI (B), otherwise it will output 'no gingivitis'. A further embodiment may use the specific value of (S) to indicate the certainty with which the indication (I) is made. This can be a probability score, whereby 0.5 is a possible threshold value, and e.g. a score S=0.8 would indicate the probability of gingivitis. Interesting options are:

Based on the score S, one can directly indicate a certainty, i.e. S=0.8 means 80% certainty of gingivitis; or
To make the indication through the definition of a range R1-R2, such that when R1<S<R2, the indication (I) will read 'inconclusive'.

A specific calculation of the score can be implemented, e.g., by means of a sigmoid function applying the following formula:

$$S = \frac{1}{1 + \exp(-(c_0 + \sum_{i=1}^{N} c_i B_i))}$$

Wherein $N$ is the number of proteins/biomarkers used. $c_0$, $c_1$, etc. are coefficients (numerical values) and $B_1, B_2$, etc. are the respective protein concentrations.

Determining of the coefficients $c_i$ can be done by a training procedure:

- Select N1 subjects with gingivitis (as identified by a dentist via the current criteria) and N2 subjects without gingivitis (having healthy gums).
- Take a saliva sample from each subject and determine the protein concentrations of a combination of biomarkers as explained above.
- Define the score S to be 1 for gingivitis, and 0 for no gingivitis (healthy gums).
- Fit the sigmoid function to the scores and protein concentration values.

Other regression or machine learning methods (linear regression, neural network, support vector machine) may be used where the score S, is high for gingivitis patients and low for the non-gingivitis/healthy controls.

In particular, such a procedure has been applied (in the Example) using a clinical study with subjects having either gingivitis or a healthy oral conditions (identified by clinical assessment by a dental professional via current criteria, e.g. American Academy of Periodontology criteria). Performance of various biomarker combinations were evaluated by means of Leave-1-out cross validation, resulting in the preferred biomarker combinations of the invention.

With reference to the aforementioned system, the invention also provides, in a further aspect, a system for assessing whether a human patient has gingivitis, the system comprising:

- detection means able and adapted to detect in a sample of saliva of the human patient the proteins:

  Alpha-1-acid glycoprotein (A1AGP), Matrix metalloproteinase-8 (MMP8), Matrix metalloproteinase-9 (MMP9) and Hemoglobin subunit beta (Hb-beta);
  as explained above, such means are known, and easily accessible to the skilled person. Typically, there is provided a container for receiving an oral sample of a subject therein, the container provided with the detection means;

- a processor able and adapted to determine from the determined concentrations of said proteins an indication of the patient having gingivitis.

Optionally, the system comprises a user interface (or a data connection to remote interface), particularly a graphical user interface (GUI), capable of presenting information; a GUI is a type of user interface that allows users to interact with electronic devices through graphical icons and visual indicators such as secondary notation, instead of text-based user interfaces, typed command labels or text navigation (none of such interface types being excluded in the present invention); GUIs are generally known, and are used typically in handheld mobile devices such as MP3 players, portable media players, gaming devices, smartphones and smaller household, office and industrial controls; as said, the interface optionally can also be chosen so as to be capable of putting in information, such as, e.g., the age of the subject, sex, BMI (Body Mass Index).

The invention also provides, either separately or as part of the aforementioned system, a kit for detecting at least two biomarkers for gingivitis in a sample of saliva of a human patient, said kit comprising one or more detection reagents for detecting the proteins:
Alpha-1-acid glycoprotein (A1AGP), Matrix metalloproteinase-8 (MMP8), Matrix metalloproteinase-9 (MMP9) and Hemoglobin subunit beta (Hb-beta).

Typically, the kit comprises four or more detection reagents, each directed to a different biomarker.

As discussed above with reference to the method of the invention, the kit may comprise more detection reagents, such as for other proteins. In a preferred embodiment the detection reagents made available in the kit consist of the detection reagents for the detection of three or four proteins making up a biomarker panel of the invention, as mentioned. In further embodiments, separate detection reagents are provided for each of the biomarker proteins present in a combination exemplified in Table 1 in the Example below.

Preferably said kits comprise a solid support, such as a chip, a microtiter plate or a bead or resin comprising said detection reagents. In some embodiments, the kits comprise mass spectrometry probes, such as ProteinChip™.

The kits may also provide washing solutions and/or detection reagents specific for either unbound detection reagent or for said biomarkers (sandwich type assay).

In an interesting aspect, the recognition of a biomarker panel of the invention is applied in monitoring the status of gingivitis in a human patient, over time.

The invention also provides a method of diagnosing whether a human patient has gingivitis, comprising detecting in a sample of saliva of the human patient the proteins:

Alpha-1-acid glycoprotein (A1AGP), Matrix metalloproteinase-8 (MMP8), Matrix metalloproteinase-9 (MMP9), and Hemoglobin subunit beta (Hb-beta).

The presence of gingivitis in the patient is typically assessed on the basis of the concentrations of said proteins in said sample. Optionally, the method of this aspect comprises the further step of treating the gingivitis in the patient. This optional treatment step can comprise the administration of known therapeutic agents or dental procedures, or a combination of therapeutic agents and dental procedures. Known therapeutic agents include the administration of antimicrobial-containing agents such as a mouthwash, chip, gel or microsphere. A typical antimicrobial agent for use in treating gingivitis is chlorhexidine. Other therapeutic agents include antibiotics, typically orally-administered antibiotics, and enzyme suppressants such as doxycycline. Known non-surgical therapeutic procedures include scaling and root planing (SRP). Known surgical procedures include surgical pocket reduction, flap surgery, gum grafts or bone grafts, although these are typically reserved for advanced periodontitis and not typically used to treat gingivitis.

The general disclosure further provides a method of detecting the proteins:

Alpha-1-acid glycoprotein (A1AGP) and at least one of Matrix metalloproteinase-8 (MMP8), Matrix metalloproteinase-9 (MMP9), Hepatocyte growth factor (HGF), Hemoglobin subunit beta (Hb-beta), and S100 calcium-binding protein A8 (S100A8); or

Hepatocyte growth factor (HGF) and at least one of the proteins Matrix metalloproteinase-8 (MMP8) and Keratin 4 (K-4); or

Matrix metalloproteinase-8 (MMP8) and at least one of the proteins Interleukin-1β (IL-1β), Keratin 4 (K-4), and Profilin;

in a human patient, comprising:

(a) obtaining a saliva sample from a human patient; and
(b) detecting whether the proteins are present in the sample by contacting the sample with one or more detection reagents for binding said proteins and detecting binding between each protein and the one or more detection reagents.

The invention will be further illustrated with reference to the following nonlimiting example.

Example

A clinical study was carried out with 74 subjects, of who 35 were diagnosed with gingivitis and 39 had healthy gums, we obtained Receiver-Operator-Characteristic Area-Under-the Curve values of >0.75 using panels containing between 2 and 4 protein biomarkers, as set out below.

ROC (Receiver-Operator-Characteristic) Area-Under-the Curve (AUC) values were obtained. Performance of various biomarker combinations were evaluated by means of logistic regression with leave-one-out cross validation (LOOCV), resulting in the preferred biomarker combinations as explained herein.

In statistics, a receiver operating characteristic curve, or ROC curve, is a graphical plot that illustrates the performance of a binary classifier system as its discrimination threshold is varied. The curve is created by plotting the true positive rate (TPR) against the false positive rate (FPR) at various threshold settings. The true-positive rate is also known as sensitivity, recall or *probability of detection* in machine learning. The false-positive rate is also known as the fall-out or *probability of false alarm* and can be calculated as (1 - specificity). The ROC curve is thus the sensitivity as a function of fall-out. In general, if the probability distributions for both detection and false alarm are known, the ROC curve can be generated by plotting for every value of the threshold, the value of the cumulative distribution function (area under the probability distribution from - ∞ to the discrimination threshold) of the detection probability on the y-axis, versus the value of the cumulative distribution function of the false-alarm probability on the x-axis. The accuracy of the test depends on how well the test separates the group being tested into those with and without the disease in question. Accuracy is measured by the area under the ROC curve. An area of 1 represents a perfect test; an area of 0.5 represents a worthless test. A guide for classifying the accuracy of a diagnostic test is the traditional academic point system:

- 0.90-1 = excellent (A)
- 0.80-0.90 = good (B)
- 0.70-0.80 = fair (C)
- 0.60-0.70 = poor (D)
- 0.50-0.60 = fail (F)

Based on the foregoing, in the results of the aforementioned clinical study, an ROC AUC value of above 0.75 is considered to represent a desirable accuracy for providing a diagnostic test in accordance with the invention.

The protein biomarkers explored are:

- MMP8
- MMP9
- IL-1$\beta$
- HGF
- Free Light Chain (FLC) $\kappa$ (kappa)
- Free light chain (FLC) $\lambda$ (lambda)
- A1AGP
- Hb-beta
- Hb-delta
- Keratin 4
- Profilin
- Pyruvate Kinase
- S100A8
- S100A9

Furthermore, in the employed logistic regression we considered as additional predictors $\kappa + \lambda$, $\kappa - \lambda$, $\kappa / \lambda$.

Additionally age was included as a predictor.

This yields a total number of 4204 possible non-redundant panels, having at most 4 protein biomarkers (panel having only age is not considered). Non-redundant here means that a panel including e.g. $\kappa + \lambda$ and $\kappa - \lambda$ as predictors is not considered, as in the logistic regression it gives the same result as the corresponding panel including $\kappa$ and $\lambda$ as predictors.

Note that not restricting the number of protein markers in a panel, yields a number of 98302 possible non-redundant panels (panel having only age is not considered) given the predictors mentioned above.

From this study 407 panels were identified that provide AUC LOOCV>0.75 for classifying gingivitis versus oral health. The preferred biomarker panels of the invention cover (at least) these 407 identified panels.

Furthermore, of these 407 panels:

- 6 have only two protein markers
- 65 have three protein markers
- 336 have four protein markers

The 6 panels containing only 2 protein markers are:

- MMP8 + A1AGP (AUC LOOCV=0.788)
- MMP9 + A1AGP (AUC LOOCV=0.788)
- HGF+Keratin 4 (AUC LOOCV= 0.775)
- MMP8 + A1AGP +Age (AUC LOOCV=0.780)
- MMP9 + A1AGP +Age (AUC LOOCV=0.766)
- HGF+Keratin 4 +Age (AUC LOOCV= 0.759)

Each of these panels is highlighted as a preferred embodiment of the invention.

(Note that these panels are actually 3 panels that may additionally include age, and for these panels age does not increase performance)

Furthermore, 14 panels are found to have AUC LOOCV>0.85. These are given by Table 1, below:

| MMP8 | IL1B | MMP9 | HGF | Age | κ | λ | κ+λ | κ/λ | κ-λ | A1AGP | Hb-beta | Hb-delta | Keratin 4 | Profilin | Pyruvate Kinase | S100A8 | S100A9 | AUC LOOCV |
|---|---|---|---|---|---|---|---|---|---|---|---|---|---|---|---|---|---|---|
| X | X |  |  |  |  |  |  |  |  | X | X |  |  |  |  |  |  | 0.860 |
| X |  | X |  |  |  |  |  |  |  | X | X |  |  |  |  |  |  | 0.854 |
| X | X |  |  | X |  |  |  |  |  | X | X |  |  |  |  |  |  | 0.851 |
| X |  |  |  |  | X |  |  |  |  | X | X |  |  |  |  |  |  | 0.857 |
|  |  | X |  |  | X |  |  |  |  | X | X |  |  |  |  |  |  | 0.867 |
| X |  |  | X | X |  |  |  |  |  | X | X |  |  |  |  |  |  | 0.853 |
|  |  | X | X | X |  |  |  |  |  | X | X |  |  |  |  |  |  | 0.862 |
| X |  |  |  |  |  |  |  |  |  | X | X |  | X |  |  |  |  | 0.888 |
|  |  | X |  |  |  |  |  |  |  | X | X |  | X |  |  |  |  | 0.874 |
| X |  |  |  | X |  |  |  |  |  | X | X |  | X |  |  |  |  | 0.880 |
|  |  | X | X |  |  |  |  |  |  | X | X |  | X |  |  |  |  | 0.862 |
|  |  | X |  |  |  |  |  |  |  | X | X |  |  |  |  |  | X | 0.851 |
|  |  |  |  |  |  |  |  |  |  | X | X |  | X |  |  |  | X | 0.857 |
|  |  |  |  | X |  |  |  |  |  | X | X |  | X |  |  |  | X | 0.853 |

Table 1

Each of the biomarker combinations in this table is highlighted as a preferred combination of the invention. It can be seen that all these panels have 4 protein markers. These results may be summarised as showing a preference for at least A1AGP and Hb-beta, and additionally at least one (preferably two) of MMP8, MMP9, and Keratin 4.

While the invention has been illustrated and described in detail in the drawings and foregoing description, such illustration and description are to be considered illustrative or exemplary and not restrictive; the invention is not limited to the disclosed embodiments. For example, it is possible to present detection reagents for different biomarkers in different units. Or, conveniently, a kit of the invention can comprise a fixed set of detection reagents for the protein biomarkers that are used in all embodiments, e.g. A1AGP, MMP8 or HGF, and optionally flexible modules comprising a detection reagent for other biomarkers such as S100A8 and/or K-4.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain features of the invention are recited in mutually different dependent claims does not indicate that a combination of these features cannot be used to advantage. Any reference signs in the claims should not be construed as limiting the scope.

In sum, we hereby disclose an *in vitro* method for assessing whether a human patient is suffering from gingivitis. The method is based on the insight to determine biomarker proteins. Accordingly, in a sample of saliva from a patient, the concentrations are measured of the proteins described herein. Based on the concentrations as measured, a value is determined reflecting the joint concentrations for said proteins. This value is compared with a threshold value reflecting in the same manner the joint concentrations associated with gingivitis. The comparison allows assessing whether the testing value is indicative of the presence of gingivitis in said patient. Thereby, typically, a testing value reflecting a joint concentration below the joint concentration reflected by the threshold value is indicative for the absence of gingivitis in said patient, and a testing value reflecting a joint concentration at or above the joint concentration reflected by the threshold value, is indicative for gingivitis in said patient.

**Claims**

1.  An *in vitro* method for assessing whether a human patient has gingivitis, wherein the method comprises:

- detecting, in a sample of saliva from said human patient, the concentrations of the proteins Alpha-1-acid glycoprotein (A1AGP), Matrix metalloproteinase-8 (MMP8), Matrix metalloproteinase-9 (MMP9) and Hemoglobin subunit beta (Hb-beta);
- determining a testing value reflecting the joint concentrations determined for said proteins;
- comparing said testing value with a threshold value reflecting in the same manner the joint concentrations associated with gingivitis, so as to assess whether the testing value is indicative for gingivitis in said patient.

2. A method according to claim 1, wherein the human patient is suspected to have gingivitis.

3. A method according to claim 1 or claim 2, wherein the age of the subject is determined and the testing value reflects the joint concentrations determined for said proteins, in combination with the age of the subject.

4. A method according to any preceding claim, wherein the threshold value is based on the concentrations determined for the proteins in one or more reference samples each sample associated with the presence of gingivitis or absence of gingivitis.

5. A method according to any of claims 1 to 3, wherein the threshold value is based on the concentrations of the proteins in a set of samples, including samples from subjects that have gingivitis and samples from subjects not having gingivitis.

6. A method according to any one of the preceding claims, wherein the proteins further comprise one or both of Hepatocyte growth factor (HGF) and S100 calcium-binding protein A8 (S100A8).

7. A method according to any one of claims 1 to 5, wherein the proteins consist of MMP8, MMP9, A1AGP and Hb-beta.

8. A method according to any one of the preceding claims, wherein the concentration values determined are arithmetically processed into a number between 0 and 1.

9. The use of the proteins Alpha-1-acid glycoprotein (A1AGP), Matrix metalloproteinase-8 (MMP8), Matrix metalloproteinase-9 (MMP9) and Hemoglobin subunit beta (Hb-beta) in a sample of saliva of a human patient, as biomarkers for assessing whether the patient has gingivitis.

10. The use according to claim 9, wherein the age of the human patient is also used as a biomarker.

11. A system for assessing whether a human patient has gingivitis, the system comprising:

    - detection means able and adapted to detect in a sample of saliva of the human patient the concentration of the proteins Alpha-1-acid glycoprotein (A1AGP), Matrix metalloproteinase-8 (MMP8), Matrix metalloproteinase-9 (MMP9) and Hemoglobin subunit beta (Hb-beta); and
    - a processor able and adapted to determine from the determined concentrations of said proteins an indication of the patient having gingivitis.

12. A system according to claim 11, further comprising a container for receiving an oral fluid sample, the container comprising the detection means.

13. A system according to claim 11 or 12, further comprising:

    - a user interface for presenting the indication to a user; and
    - a data connection between the processor and the user interface for transferring the indication from the processor to the user interface.

14. A system according to any one of claims 11 to 13, wherein the processor is enabled to function by means of an internet-based application.

15. A system according to any of claims 11 to 14, wherein the interface is capable of putting in information on the age of the subject and the processor is able and adapted to determine from the determined concentrations, an indication that the patient has gingivitis.

16. A kit for detecting at least four biomarkers for gingivitis in a sample of saliva of a human patient, said kit comprising detection reagents for detecting Alpha-1-acid glycoprotein (A1AGP), Matrix metalloproteinase-8 (MMP8), Matrix metalloproteinase-9 (MMP9) and Hemoglobin subunit beta (Hb-beta).

17. A kit according to claim 16, wherein the detection reagents are contained on a solid support.

18. A method of diagnosing whether a human patient has gingivitis, comprising detecting in a sample of saliva of the human patient the proteins:
Alpha-1-acid glycoprotein (A1AGP), Matrix metalloproteinase-8 (MMP8), Matrix metalloproteinase-9 (MMP9) and Hemoglobin subunit beta (Hb-beta);
and assessing the presence of gingivitis in the patient on the basis of the concentrations of said proteins in said sample.

**Patentansprüche**

1. *In-vitro*-Verfahren zum Beurteilen, ob ein menschlicher Patient Gingivitis hat, wobei das Verfahren Folgendes umfasst:

- Nachweisen, in einer Speichelprobe des menschlichen Patienten, der Konzentrationen der Proteine Alpha-1-saures Glykoprotein (A1AGP), Matrix-Metalloproteinase-8 (MMP8), Matrix-Metalloproteinase-9 (MMP9) und Hämoglobin-Untereinheit Beta (Hb-beta);
- Bestimmen eines Testwerts, der die gemeinsamen Konzentrationen, die für die Proteine bestimmt werden, widerspiegelt;
- Vergleichen des Testwerts mit einem Schwellenwert, der in gleicher Weise die Gingivitis zugeordneten gemeinsamen Konzentrationen widerspiegelt, um zu beurteilen, ob der Testwert auf Gingivitis bei dem Patienten hinweist.

2. Verfahren nach Anspruch 1, wobei bei dem menschlichen Patienten der Verdacht besteht, dass er Gingivitis hat.

3. Verfahren nach Anspruch 1 oder 2, wobei das Alter des Probanden bestimmt wird und der Testwert die gemeinsamen Konzentrationen, die für die Proteine bestimmt werden, in Kombination mit dem Alter des Probanden widerspiegelt.

4. Verfahren nach einem vorstehenden Anspruch, wobei der Schwellenwert auf den Konzentrationen basiert, die für die Proteine in einer oder mehreren Referenzproben bestimmt werden, wobei jede Probe dem Vorhandensein von Gingivitis oder dem Fehlen von Gingivitis zugeordnet ist.

5. Verfahren nach einem der Ansprüche 1 bis 3, wobei der Schwellenwert auf den Konzentrationen der Proteine in einem Satz von Proben basiert, der Proben von Subjekten, die Gingivitis haben, und Proben von Subjekten, die keine Gingivitis haben, beinhaltet.

6. Verfahren nach einem der vorstehenden Ansprüche, wobei die Proteine weiter eines oder beide von Hepatozyten-Wachstumsfaktor (HGF) und S100-Calcium-bindendem Protein A8 (S100A8) umfassen.

7. Verfahren nach einem der Ansprüche 1 bis 5, wobei die Proteine aus MMP8, MMP9, A1AGP und Hb-beta bestehen.

8. Verfahren nach einem der vorstehenden Ansprüche, wobei die bestimmten Konzentrationswerte arithmetisch zu einer Zahl zwischen 0 und 1 verarbeitet werden.

9. Verwendung der Proteine Alpha-1-saures Glykoprotein (A1AGP), Matrix-Metalloproteinase-8 (MMP8), Matrix-Metalloproteinase-9 (MMP9) und Hämoglobin-Untereinheit Beta (Hb-beta) in einer Speichelprobe eines menschlichen Patienten als Biomarker zum Beurteilen, ob der Patient Gingivitis hat.

10. Verwendung nach Anspruch 9, wobei das Alter des menschlichen Patienten auch als ein Biomarker verwendet wird.

11. System zum Beurteilen, ob ein menschlicher Patient Gingivitis hat, wobei das System Folgendes umfasst:

- Erfassungsmittel, die im Stande und dazu ausgelegt sind, in einer Speichelprobe des menschlichen Patienten die Konzentration der Proteine Alpha-1-saures Glykoprotein (A1AGP), Matrix-Metalloproteinase-8 (MMP8), Matrix-Metalloproteinase-9 (MMP9) und Hämoglobin-Untereinheit Beta (Hb-beta) zu Nachweisen; und
- einen Prozessor, der im Stande und dazu ausgelegt ist, aus den bestimmten Konzentrationen der Proteine einen Hinweis darauf zu gewinnen, ob der Patient Gingivitis hat.

12. System nach Anspruch 11, das weiter einen Behälter zum Aufnehmen einer Mundflüssigkeitsprobe umfasst, wobei der Behälter die Erfassungsmittel umfasst.

13. System nach Anspruch 11 oder 12, weiter umfassend:

   - eine Benutzerschnittstelle zum Darstellen des Hinweises für einen Benutzer; und
   - eine Datenverbindung zwischen dem Prozessor und der Benutzerschnittstelle zum Übertragen des Hinweises von dem Prozessor an die Benutzerschnittstelle.

14. System nach einem der Ansprüche 11 bis 13, wobei der Prozessor dazu fähig ist, mittels einer internetbasierten Anwendung zu arbeiten.

15. System nach einem der Ansprüche 11 bis 14, wobei die Oberfläche im Stande ist, Informationen über das Alter des Subjekts einzugeben, und der Prozessor im Stande und dazu ausgelegt ist, aus den bestimmten Konzentrationen einen Hinweis darauf zu bestimmen, dass der Patient Parodontitis hat.

16. Kit zum Nachweisen von mindestens vier Biomarkern für Gingivitis in einer Speichelprobe eines menschlichen Patienten, wobei das Kit Nachweisreagenzien zum Nachweisen von Alpha-1-saurem Glykoprotein (A1AGP), Matrix-Metalloproteinase-8 (MMP8), Matrix-Metalloproteinase-9 (MMP9) und Hämoglobin-Untereinheit Beta (Hb-beta) umfasst.

17. Kit nach Anspruch 16, wobei die Nachweisreagenzien auf einem festen Träger enthalten sind.

18. Verfahren zum Diagnostizieren, ob ein menschlicher Patient Gingivitis hat, umfassend Nachweisen der folgenden Proteine in einer Speichelprobe des menschlichen Patienten:
Alpha-1-saures Glykoprotein (A1AGP), Matrix-Metalloproteinase-8 (MMP8), Matrix-Metalloproteinase-9 (MMP9) und Hämoglobin-Untereinheit Beta (Hb-beta); und Beurteilen des Vorhandenseins von Gingivitis beim Patienten auf der Grundlage der Konzentrationen der Proteine in der Probe.

**Revendications**

1. Procédé *in vitro* d'évaluation de la présence d'une gingivite chez un patient humain, dans lequel le procédé comprend :

   - la détection, dans un échantillon de salive provenant dudit patient humain, des concentrations des protéines Alpha-1-glycoprotéine acide (A1AGP), métalloprotéinase matricielle-8 (MMP8), métalloprotéinase matricielle-9 (MMP9) et sous-unité bêta de l'hémoglobine (Hb-bêta) ;
   - la détermination d'une valeur de test reflétant les concentrations conjointes déterminées pour lesdites protéines ;
   - la comparaison de ladite valeur de test avec une valeur seuil reflétant de la même manière les concentrations conjointes associées à la gingivite, afin d'évaluer si la valeur de test est indicative d'une gingivite chez ledit patient.

2. Procédé selon la revendication 1, dans lequel le patient humain est suspecté de souffrir d'une gingivite.

3. Procédé selon la revendication 1 ou la revendication 2, dans lequel l'âge du sujet est déterminé et la valeur de test reflète les concentrations conjointes déterminées pour lesdites protéines, en combinaison avec l'âge du sujet.

4. Procédé selon l'une quelconque des revendications précédentes, dans lequel la valeur seuil est basée sur les concentrations déterminées pour les protéines dans un ou plusieurs échantillons de référence, chaque échantillon étant associé à la présence ou à l'absence de gingivite.

5. Procédé selon l'une quelconque des revendications 1 à 3, dans lequel la valeur seuil est basée sur les concentrations des protéines dans un ensemble d'échantillons, incluant des échantillons provenant de sujets atteints de gingivite et des échantillons provenant de sujets non atteints de gingivite.

6. Procédé selon l'une quelconque des revendications précédentes, dans lequel les protéines comprennent en outre le facteur de croissance des hépatocytes (HGF) et/ou la protéine de liaison au calcium S100 A8 (S100A8).

7. Procédé selon l'une quelconque des revendications 1 à 5, dans lequel les protéines sont constituées de MMP8, MMP9, A1AGP et Hb-beta.

8. Procédé selon l'une quelconque des revendications précédentes, dans lequel les valeurs de concentration déterminées sont traitées arithmétiquement en un nombre compris entre 0 et 1.

9. Utilisation des protéines Alpha-1-glycoprotéine acide (A1AGP), métalloprotéinase matricielle-8 (MMP8), métalloprotéinase matricielle-9 (MMP9) et sous-unité bêta de l'hémoglobine (Hb-bêta) dans un échantillon de salive d'un patient humain, comme biomarqueurs pour évaluer si le patient souffre de gingivite.

10. Utilisation selon la revendication 9, dans laquelle l'âge du patient humain est également utilisé comme biomarqueur.

11. Système permettant d'évaluer si un patient humain souffre de gingivite, le système comprenant :

- des moyens de détection capables et adaptés pour détecter dans un échantillon de salive du patient humain la concentration des protéines suivantes : alpha-1 glycoprotéine acide (A1AGP), métalloprotéinase matricielle-8 (MMP8), métalloprotéinase matricielle-9 (MMP9) et sous-unité bêta de l'hémoglobine (Hb-bêta) ; et
- un processeur capable et adapté pour déterminer à partir des concentrations déterminées desdites protéines une indication du patient atteint de gingivite.

12. Système selon la revendication 11, comprenant en outre un récipient destiné à recevoir un échantillon de liquide buccal, le récipient comprenant les moyens de détection.

13. Système selon la revendication 11 ou 12, comprenant en outre :

- une interface utilisateur pour présenter l'indication à un utilisateur ; et
- une connexion de données entre le processeur et l'interface utilisateur pour transférer l'indication du processeur à l'interface utilisateur.

14. Système selon l'une quelconque des revendications 11 à 13, dans lequel le processeur est capable de fonctionner au moyen d'une application basée sur Internet.

15. Système selon l'une quelconque des revendications 11 à 14, dans lequel l'interface est capable de saisir des informations sur l'âge du sujet et le processeur est capable et adapté pour déterminer, à partir des concentrations déterminées, une indication que le patient souffre de gingivite.

16. Kit permettant de détecter au moins quatre biomarqueurs de la gingivite dans un échantillon de salive d'un patient humain, ledit kit comprenant des réactifs de détection pour la détection de l'alpha-1-glycoprotéine acide (A1AGP), de la métalloprotéinase matricielle-8 (MMP8), de la métalloprotéinase matricielle-9 (MMP9) et de la sous-unité bêta de l'hémoglobine (Hb-bêta).

17. Kit selon la revendication 16, dans lequel les réactifs de détection sont contenus sur un support solide.

18. Procédé de diagnostic de la gingivite chez un patient humain, comprenant la détection dans un échantillon de salive du patient humain des protéines :
Alpha-1-glycoprotéine acide (A1AGP), métalloprotéinase matricielle-8 (MMP8), métalloprotéinase matricielle-9 (MMP9) et sous-unité bêta de l'hémoglobine (Hb-bêta) ; et évaluation de la présence de gingivite chez le patient sur la base des concentrations desdites protéines dans ledit échantillon.

FIG. 1

**EP 3 775 917 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014037924 A

**Non-patent literature cited in the description**

- **RAMSEIER et al.** *J Periodontol.*, March 2009, vol. 80 (3), 436-46